# FASCICULE DE BREVET EUROPEEN

(11) **EP 4 458 394 B1**
(45) Date de publication et mention de la délivrance du brevet: **21.01.2026**
(21) Numéro de dépôt: 24169408.2
(22) Date de dépôt: 10.04.2024
(51) Int. Cl.: A61M 16/00, A61M 16/10, A61M 16/12, A61M 16/20

(54) **APPAREIL DE DÉLIVRANCE DE MONOXYDE D'AZOTE GAZEUX EN MODE PROPORTIONNEL OU PULSÉ**
VORRICHTUNG ZUR ABGABE VON GASFÖRMIGEM STICKSTOFFMONOXID IM PROPORTIONAL- ODER PULSBETRIEB
APPARATUS FOR DELIVERING GASEOUS NITRIC OXIDE IN PROPORTIONAL OR PULSED MODE

(30) Priorité: 04.05.2023 FR 2304471
(43) Date de publication de la demande: 06.11.2024
(73) Titulaire: INOSYSTEMS, 92160 Antony (FR)
(72) Inventeur: MARCHAL, Frederic, 92160 Antony (FR); BOULANGER, Thierry, 92160 Antony (FR); BLANDIN, Yann, 92220 Bagneux (FR); SCHMITT, Mary, 92220 Bagneux (FR)
(74) Mandataire: Air Liquide

(56) Documents cités:
- EP-A1- 3 466 473
- EP-B1- 0 886 532
- FR-A1- 2 911 281
- US-A1- 2020 398 019
- US-A1- 2021 308 411

## Description

L'invention concerne un dispositif ou appareil de fourniture ou délivrance de monoxyde d'azote gazeux (NO), et une installation d'administration de gaz thérapeutique à base de NO à un patient comprenant un tel dispositif ou appareil de fourniture de NO.

Le monoxyde d'azote inhalé ou NOi est un médicament gazeux couramment utilisé pour traiter des patients souffrant d'hypertension artérielle pulmonaire aiguë, en particulier les vasoconstrictions pulmonaires chez l'adulte ou l'enfant, y compris les nouveau-nés (PPHN), comme décrit par exemple par EP-A-560928 ou EP-A-1516639.

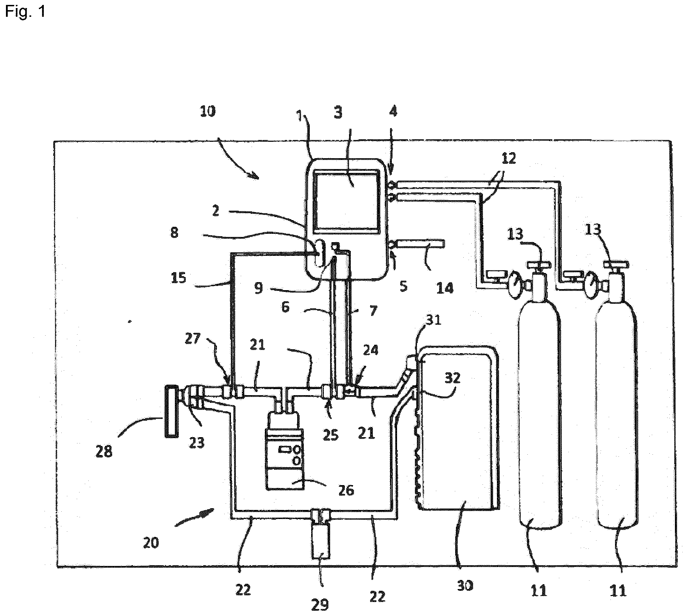

Une installation de mise en œuvre d'un traitement par NOi, couramment appelée installation d'administration de gaz thérapeutique à base de NO à un patient ou plus simplement installation d'administration de NO, comprend classiquement une ou des bouteilles de mélange NO/N₂ alimentant un dispositif de fourniture de NO qui délivre le mélange NO/N₂ à débit contrôlé, un ventilateur médical, c'est-à-dire un appareil d'assistance respiratoire, pour fournir un gaz respiratoire contenant au moins 20 à 21% vol. d'oxygène environ, tel un mélange O₂/N₂ ou de l'air, auquel est ajouté le NO sous forme d'un mélange NO/N₂, des éléments de circuit, par exemple un ou plusieurs conduits de gaz flexibles, pour acheminer les flux gazeux entre ces différents équipements et jusqu'au patient, et une interface respiratoire, telle une sonde d'intubation trachéale, pour fournir le mélange gazeux contenant le NO au patient. On peut prévoir aussi un humidificateur de gaz pour humidifier le mélange gazeux avant son administration au patient. Une telle installation d'administration de NO est schématisée en Fig. 1.

Une telle installation d'administration de NO est utilisée en milieu hospitalier pour administrer le traitement par NOi et ainsi soigner les patients ayant besoin d'inhaler du NO pour traiter leur hypertension artérielle pulmonaire. Des exemples de telles installations d'administration de NO sont donnés par les documents EP 3 466 473 A1, FR 2 911 281 A1, EP-A-3821929, WO-A-2012/094008, US-A-2015/320951, US-A-2015/273175, JP-AH11192303, WO-A-02/40914 et US-A-2003/116159.

Afin de délivrer efficacement le gaz au(x) patient(s), l'appareil de fourniture de NO qui fait partie de l'installation d'administration de NO sont généralement équipés d'actionneurs de type électrovanne proportionnelle, comme enseigné par EP-A-659445, ou d'électrovannes tout ou rien, voire une combinaison des deux comme décrit par EP-A-375671, qui permettent de générer différentes valeurs de débits en fonction notamment de la concentration de NO à délivrer fixée par le personnel soignant et de la concentration des bouteilles. En particulier, plus la concentration dans les bouteilles est élevée, plus le débit de NO à délivrer devra être faible.

Dès lors, il convient d'utiliser des actionneurs extrêmement précis dans les faibles gammes de débits de NO, notamment lors d'un traitement d'enfant ou de nouveau-né, mais permettant aussi de délivrer des débits plus élevés pour les traitements d'adultes qui requièrent du NO à plus haute concentration.

Or, cela conduit à des problèmes et inconvénients. En effet, afin de pouvoir couvrir les différentes plages de débit requises, il est nécessaire de multiplier les actionneurs, typiquement en les montant en parallèle, ce qui engendre une complexification du circuit de gaz interne de l'appareil de fourniture de NO et forcément de son coût.

Autrement dit, il existe un besoin pour un appareil ou dispositif de fourniture de NO, typiquement de mélange gazeux NO/N₂, qui ne présente pas toute ou partie des inconvénients et problèmes susmentionnés, lequel soit adapté au traitement de patients souffrant en particulier d'hypertension artérielle pulmonaire.

Une solution selon l'invention concerne un appareil ou dispositif de fourniture (i.e. de délivrance) de NO, typiquement d'un mélange gazeux NO/N₂, comprenant :
- un circuit de gaz principal pour acheminer un mélange gazeux NO/N₂,
- des moyens de contrôle de débit comprenant (au moins) une électrovanne principale agencée sur ledit circuit de gaz principal, et
- des moyens de pilotage configurés pour commander au moins ladite au moins une électrovanne principale pour contrôler le débit de mélange gazeux NO/N₂ traversant ladite au moins une électrovanne principale.

De plus, l'électrovanne principale est une électrovanne proportionnelle ayant des degrés d'ouverture compris entre 0 et 100%, et est configurée pour fonctionner dans au moins deux plages de fonctionnement données comprenant au moins :
- une première plage de fonctionnement correspondant à des degrés d'ouverture de l'électrovanne principale compris entre 0 et x% avec : 0 < x% ≤ 10%, et
- une deuxième plage de fonctionnement correspondant à des degrés d'ouverture de l'électrovanne principale supérieurs à x% (i.e. >x%).

Par ailleurs, les moyens de pilotage sont configurés pour commander ladite électrovanne principale
pour opérer une fourniture de gaz de manière différente selon la plage de fonctionnement considérée, à savoir :
▪ en mode pulsé (i.e. en modulation d'impulsions) dans la première plage de fonctionnement de manière à délivrer des impulsions (IG) gazeuses successives, et
▪ en mode proportionnel dans la deuxième plage de fonctionnement,

De plus, l'appareil ou dispositif de fourniture de NO comprend en outre un circuit (ou une ligne) de bipasse venant se raccorder fluidiquement au circuit de gaz principal, en amont et en aval des moyens de contrôle de débit et comprenant des moyens de contrôle de débit secondaires comprenant une électrovanne secondaire de type tout ou rien et un dispositif à débit fixe.

Selon le mode de réalisation considéré, l'appareil ou dispositif de fourniture, i.e. de délivrance, de NO de l'invention peut comprendre l'une ou plusieurs des caractéristiques suivantes :
- la première plage de fonctionnement est non-linéaire.
- la deuxième plage de fonctionnement est linéaire.
- les moyens de pilotage sont configurés pour commander ladite électrovanne principale en mode proportionnel dans la deuxième plage de fonctionnement, en opérant une ouverture ou une fermeture progressivement (i.e. proportionnelle) de l'électrovanne principale pour obtenir le débit de gaz désiré.
- selon le mode de réalisation considéré, la première plage de fonctionnement correspond à des degrés d'ouverture de l'électrovanne principale compris entre 0 et x% avec : 0 < x% ≤ 8%, alternativement 0 < x% ≤ 5%, alternativement 0 < x% ≤ 4%, alternativement 0 < x% ≤ 3%, ou alternativement 0 < x% ≤ 2%.
- les moyens de pilotage sont configurés pour fixer ou ajuster une durée ou une amplitude de chaque impulsion gazeuse délivrée en mode pulsé, pour contrôler le débit de gaz traversant ladite électrovanne principale.
- les moyens de pilotage sont configurés pour ajuster, pour chaque impulsion, la durée d'impulsion en contrôlant la durée d'ouverture de l'électrovanne proportionnelle. Lorsque la durée est variable, l'amplitude de chaque impulsion est fixe.
- les moyens de pilotage sont configurés pour ajuster, pour chaque impulsion, l'amplitude d'impulsion en contrôlant le degré d'ouverture de l'électrovanne proportionnelle. Lorsque l'amplitude est variable, la durée de chaque impulsion est fixe.
- l'électrovanne principale, i.e. proportionnelle, est traversée par un passage intérieur en communication fluidique avec le circuit de gaz principal.
- lorsque l'électrovanne principale est au moins partielle ouverte (i.e. degré d'ouverture non nul), le mélange gazeux NO/N₂ circule dans le passage intérieur de l'électrovanne principale soit sous forme d'un flux gazeux continu, soit sous forme d'impulsions gazeuses.
- la durée d'ouverture de l'électrovanne proportionnelle, c'est-à-dire la durée d'impulsion, est comprise entre 5 et 200 msec environ.
- en mode pulsé, les moyens de pilotage sont configurés pour commander l'électrovanne proportionnelle pour délivrer des impulsions gazeuses successives où chaque impulsion gazeuse présente une durée d'impulsion non-nulle pendant lequel l'électrovanne proportionnelle est en position d'ouverture partielle avec un degré d'ouverture x%, tel que x%<10%, de sorte d'autoriser le passage d'une quantité de mélange NO/N₂ donnée pendant ladite durée d'impulsion.
- les impulsions gazeuses sont séparées les unes des autres par des temps de fermeture pendant lesquels l'électrovanne proportionnelle est en position fermée, c'est-à-dire présentant un degré d'ouverture x%, tel que x%=0% (i.e. état de fermeture).
- le temps de fermeture est compris entre 0 et 195 ms, de préférence un temps de fermeture non-nul.
- les moyens de contrôle de débit permettent de contrôler le flux gazeux (i.e. NO/N₂) dans le circuit de gaz principal.
- le circuit de gaz principal relie fluidiquement au moins un port d'entrée de NO à au moins un orifice de sortie de NO pour acheminer le mélange gazeux NO/N₂ dudit au moins un port d'entrée de NO audit au moins un orifice de sortie de NO.
- le circuit de secours comprend des moyens de contrôle de débit secondaires pour contrôler le flux gazeux (i.e. NO/N₂) dans le circuit de secours.
- les moyens de pilotage sont configurés pour commander ladite au moins une électrovanne principale des moyens de contrôle de débit et/ou ladite au moins une électrovanne secondaire des moyens de contrôle de débit secondaires pour contrôler le débit de mélange NO/N₂ traversant ladite au moins une électrovanne principale et/ou ladite au moins une électrovanne secondaire.
- les moyens de contrôle de débit secondaires comprenant au moins une électrovanne secondaire, sont agencés sur ledit circuit de bipasse.
- l'électrovanne secondaire est de type tout ou rien (TOR).
- l'électrovanne TOR est configurée pour adopter uniquement deux positions de fonctionnement comprenant une position ouverte laissant passer le flux de mélange NO/N₂ et une position fermée interdisant tout passage de flux de mélange NO/N₂.
- les moyens de pilotage sont configurés pour commander ladite au moins une électrovanne secondaire, i.e. électrode TOR, en mode pulsé pour passer alternativement ladite au moins une électrovanne secondaire en position d'ouverture (i.e. en ouverture maximale) et en position de fermeture de manière à délivrer le mélange NO/N₂ sous forme d'impulsions gazeuses successives.
- chaque impulsion gazeuse comprend un temps d'impulsion (di) de durée non-nulle pendant lequel ladite au moins une électrovanne secondaire est en position d'ouverture et autorise le passage d'une quantité de mélange NO/N₂ correspondant au débit fixe (Q_{fixe}) prédéterminé de mélange NO/N₂ délivré par le dispositif à débit fixe pendant le temps d'impulsion (di) de l'impulsion gazeuse considérée.
- il comprend en outre un dispositif à débit fixe agencé sur le circuit de bipasse délivrant un débit fixe (Q_{fixe}) prédéterminé de mélange NO/N₂.
- le dispositif à débit fixe comprend un orifice calibré.
- le dispositif à débit fixe comprend un dispositif à orifice calibré délivrant le débit fixe prédéterminé (Qfixe).
- typiquement, le débit fixe prédéterminé (Q_{fixe}) dans le circuit de secours est compris entre 0.1 et 2 L/min, de préférence entre 0.1 et 1 L/min, typiquement de l'ordre de 0.5 I/min.
- le temps d'impulsion (di) de chaque impulsion gazeuse est de durée variable non-nulle, de préférence compris entre 5 msec et 200 msec environ.
- les impulsions gazeuses sont séparées les unes des autres par des temps de fermeture (df) pendant lesquels l'électrovanne principale proportionnelle et/ou secondaire électrovanne sont en position fermée.
- les temps de fermeture (df) ont des durées égales ou variables.
- typiquement, les temps de fermeture (df) ont des durées comprises entre supérieures ou égale à 0 msec et pouvant aller jusqu'à 195 msec.
- il comprend des moyens de mémorisation configurés pour mémoriser le débit fixe (Q_{fixe}) prédéterminé délivré par le dispositif à débit fixe.
- de préférence les moyens de mémorisation sont intégrés aux moyens de pilotage.
- les moyens de mémorisation comprennent une mémoire informatique, par exemple une mémoire flash, RAM ou autre.
- le dispositif à débit fixe est agencé sur ledit circuit de bipasse, en aval de ladite au moins une électrovanne secondaire.
- les moyens de pilotage sont configurés pour déterminer la quantité de mélange NO/N₂ souhaitée à partir du débit de gaz fourni par un ventilateur médical, de la concentration souhaité et de la teneur en NO du mélange NO/ N₂.
- les moyens de pilotage comprennent au moins un (micro)processeur.
- ledit au moins un (micro)processeur est agencé sur une carte électronique.
- ledit au moins un (micro)processeur met en œuvre au moins un algorithme.
- le circuit de gaz principal comprend ladite au moins une électrovanne principale et au moins un capteur de débit.
- ledit au moins un capteur de débit est agencé en amont de ladite au moins une électrovanne principale.
- ledit au moins un capteur de débit est raccordé électriquement aux moyens de pilotage.
- ladite au moins une électrovanne principale est une électrovanne proportionnelle commandée par les moyens de pilotage.
- il comprend un contrôleur de débit massique ou Mass Flow Controler (MFC) comprenant l'électrovanne principale et le capteur de débit agencés sur le circuit de gaz principal.
- le circuit de gaz principal comprend une ligne principale de NO.
- ladite au moins une électrovanne principale et ledit au moins un capteur de débit sont agencés sur la ligne principale de NO.
- la partie amont de ligne principale de NO se ramifie en deux tronçons parallèles comprenant chacun un orifice d'entrée de gaz pour recevoir un mélange NO/N₂.
- la ligne de bipasse est raccordée fluidiquement à la ligne principale de NO, en amont et en aval desdits moyens de contrôle de débit de manière à bipasser lesdits moyens de contrôle de débit.
- le circuit de bipasse comprend en outre un dispositif détendeur de gaz agencé en amont de ladite au moins une électrovanne secondaire.

L'invention concerne aussi une installation de fourniture de gaz, i.e. un gaz contenant du NO et de l'oxygène, à un patient comprenant :
- au moins une source de mélange NO/N₂, typiquement une ou plusieurs bouteilles de gaz sous pression contenant le mélange NO/N₂,
- un appareil de fourniture de NO selon l'invention raccordé fluidiquement à ladite au moins une source de mélange NO/N₂,
- un ventilateur médical configuré pour fournir un gaz respiratoire contenant de l'oxygène, typiquement au moins 20% environ d'oxygène, tel de l'air ou un mélange N₂/O₂, par exemple au moins 21% environ d'oxygène,
- un circuit patient auquel sont raccordés fluidiquement l'appareil de fourniture de NO selon l'invention et le ventilateur médical pour alimenter ledit circuit patient en ledit mélange gazeux NO/N₂ et en ledit gaz respiratoire contenant de l'oxygène,
- et un capteur de débit agencé sur le circuit patient et configuré pour déterminer (i.e. mesurer) et fournir aux moyens de pilotage du dispositif de délivrance de NO, au moins un signal de mesure représentatif du débit gazeux au sein du circuit patient, à savoir un (ou des) signal ou une(ou des) mesure de débit ou de pression.

Selon le mode de réalisation considéré, l'installation de fourniture de gaz de l'invention peut comprendre l'une ou plusieurs des caractéristiques suivantes :
- le circuit patient comprend un module d'injection de NO alimenté en mélange NO/N₂ par l'appareil de fourniture de NO, de préférence via une ligne ou un conduit d'injection de NO, telle une conduite de gaz flexible.
- le capteur de débit est agencé sur une branche inspiratoire du circuit patient, et est relié électriquement aux moyens de pilotage, via une ou de liaisons électriques (e.g. câbles ou analogues).
- le capteur de débit (i.e. servant à mesurer le débit du gaz respiratoire fourni par le ventilateur) est agencé sur le circuit patient entre le ventilateur médical et le module d'injection de NO.
- selon un mode de réalisation, le capteur de débit est du type capteur de débit massique ou analogue. Toutefois, un autre type de capteur peut être utilisé dès lors qu'il permet d'obtenir une mesure directe ou indirecte du débit.
- le capteur de débit est relié électriquement aux moyens de pilotage, via une ou des liaisons électriques, tels des câbles ou analogues.
- l'appareil de fourniture de NO est alimenté en mélange gazeux NO/N₂ formé de NO et d'azote provenant de la ou des sources de mélange NO/N₂
- la ligne d'acheminement de NO de l'appareil de fourniture de NO achemine le mélange gazeux NO/N₂.
- le mélange gazeux NO/N₂ provenant de la (ou les) source de mélange NO/N₂ contient entre 100 et 2000 ppmv de NO, typiquement moins de 1000 ppmv de NO, le reste étant de l'azote (et éventuellement des impuretés inévitables).
- l'appareil de fourniture de NO comprend un boitier, dans lequel sont agencés le circuit principal, le circuit de bipasse, les moyens de pilotage, les électrovannes principale et secondaire, et d'autres composants.
- des moyens d'alimentation électrique alimentant en courant électrique les composants nécessitant de l'énergie électrique pour fonctionner, notamment l'appareil de fourniture de NO et le ventilateur médical, tels des moyens de raccordement au secteur (110/220V) et/ou une (des) batterie ou analogue.
- le ventilateur médical comprend une soufflante motorisée (i.e. aussi appelée turbine, compresseur ou analogue) délivrant le gaz respiratoire, typiquement de l'air ou un mélange oxygène/azote, voire de l'oxygène pur.
- la (les) source de NO contient un mélange gazeux NO/N₂ contenant entre 100 et 2000 ppmv de NO, le reste étant de l'azote (N₂), conditionné à une pression comprise entre 10 et 250 bar abs, typiquement à plus de 100 bar abs (avant début de soutirage), de préférence entre 100 et 1000 ppmv de NO, le reste étant de l'azote (N₂).
- la (les) source de NO comprend une (ou des) bouteille de gaz sous pression.
- la source de NO comprend une (ou des) bouteille de gaz ayant une contenance comprise entre 0,5 et 50 L (équivalent en eau).
- la bouteille de gaz comprend un corps cylindrique en acier ou en alliage d'aluminium et est équipée d'un robinet simple (sans détendeur) ou à détendeur intégré ou RDI, de préférence un RDI protégé par un capotage de protection, par exemple en métal ou polymère.
- le circuit patient comprend une branche inspiratoire et une branche expiratoire.
- le circuit patient comprend des conduites flexibles formant la branche inspiratoire et/ou la branche expiratoire, typiquement des tuyaux en polymère.
- la branche inspiratoire et la branche expiratoire, e.g. des conduites flexibles, sont raccordées à une pièce de jonction, telle une pièce en Y.
- la branche inspiratoire et/ou la branche expiratoire sont reliées fluidiquement à une interface respiratoire patient, de préférence via la pièce de jonction.
- l'interface respiratoire patient comprend une sonde d'intubation trachéale ou masque respiratoire.
- la branche inspiratoire et la branche expiratoire comprennent des conduites flexibles, par exemple en polymère.
- la branche inspiratoire et la branche expiratoire sont en outre fluidiquement raccordées à, respectivement, des orifices de sortie et d'entrée du ventilateur médical.
- la branche inspiratoire du circuit patient peut comprendre un humidificateur de gaz agencé en aval du module d'injection de NO de manière à pouvoir humidifier le gaz avant son administration par inhalation au patient.
- la concentration ou posologie de NO dans le mélange gazeux final, c'est-à-dire le gaz qui est inhalé par le patient, après mélange du mélange NO/N₂ avec le gaz respiratoire contenant de l'oxygène provenant du ventilateur, tel de l'air ou un mélange O₂/N₂ (>20%vol. environ), est comprise entre 1 et 80 ppm en volume (ppmv), en fonction de la population traitée, i.e. nouveau-nés ou adultes, de l'état du patient et/ou de la maladie à traiter.

L'appareil de fourniture de NO et/ou l'installation de fourniture de gaz de l'invention sont particulièrement bien adaptés à une utilisation dans le cadre d'une méthode de traitement thérapeutique mettant en œuvre une administration par inhalation, en particulier via une sonde d'intubation trachéale, d'un mélange gazeux comprenant de 1 à 80 ppmv de NO et au moins 20%vol. environ d'oxygène, typiquement de l'ordre de 10 à 20 ppmv de NO, à un ou des patients (e.g. adultes, enfants, adolescents ou nouveau-nés), en ayant besoin, typiquement des patients (i.e. des personnes humaines) souffrant d'hypertension pulmonaire et/ou d'hypoxie susceptibles d'engendrer des vasoconstrictions pulmonaires ou analogues, par exemple causés par des pathologies ou troubles pulmonaires de type PPHN (hypertension pulmonaire persistante du nouveau-né) ou SDRA (syndrome de détresse respiratoire aigüe), ou encore engendrés par une opération de chirurgie cardiaque avec mise du patient sous circulation sanguine extracorporelle (CEC).

Dans le cadre de l'invention :
- « ppmv » signifie partie par million en volume,
- « %vol. » » signifie pourcentage en volume.
- « NO » désigne le monoxyde d'azote.
- « N₂ » désigne l'azote.
- « O₂ » désigne l'oxygène.
- Par « capteur de débit », on entend un capteur du type mesurant et fournissant un ou des signaux ou mesures de débit proprement-dits ou du type mesurant et fournissant un ou des signaux ou mesures de pression qui sont converties ensuite en débit par les moyens de pilotage.
- Tous les termes « moyen de » sont considérés comme totalement équivalents et substituables par les termes « dispositif de », par exemple les termes « moyens de pilotage » peuvent être remplacés par « dispositif de pilotage », les termes « moyens de mesure » peuvent être remplacés par « dispositif de mesure », les « moyens de contrôle » peuvent être remplacés par « dispositif de contrôle »...

L'invention va maintenant être mieux comprise grâce à la description détaillée suivante, faite à titre illustratif mais non limitatif, en référence aux figures annexées parmi lesquelles :
- Fig. 1 schématise une installation d'administration de gaz thérapeutique à base de NO à un patient intégrant un dispositif de fourniture de NO selon l'invention,
- Fig. 2 schématise le fonctionnement du dispositif de fourniture de NO selon l'invention,
- Fig. 3 schématise les impulsions gazeuses successives résultant du contrôle en mode pulsé de l'électrovanne secondaire du dispositif de fourniture de NO de Fig. 2,
- Fig. 4 schématise les impulsions gazeuses successives résultant d'un contrôle de la largeur de chaque impulsion gazeuse en mode pulsé, lors d'un fonctionnement de l'électrovanne principale dans sa plage de fonctionnement non-linéaire, et
- Fig. 5 schématise les impulsions gazeuses successives résultant d'un contrôle de l'amplitude de chaque impulsion gazeuse en mode pulsé, lors d'un fonctionnement de l'électrovanne principale dans sa plage de fonctionnement non-linéaire.

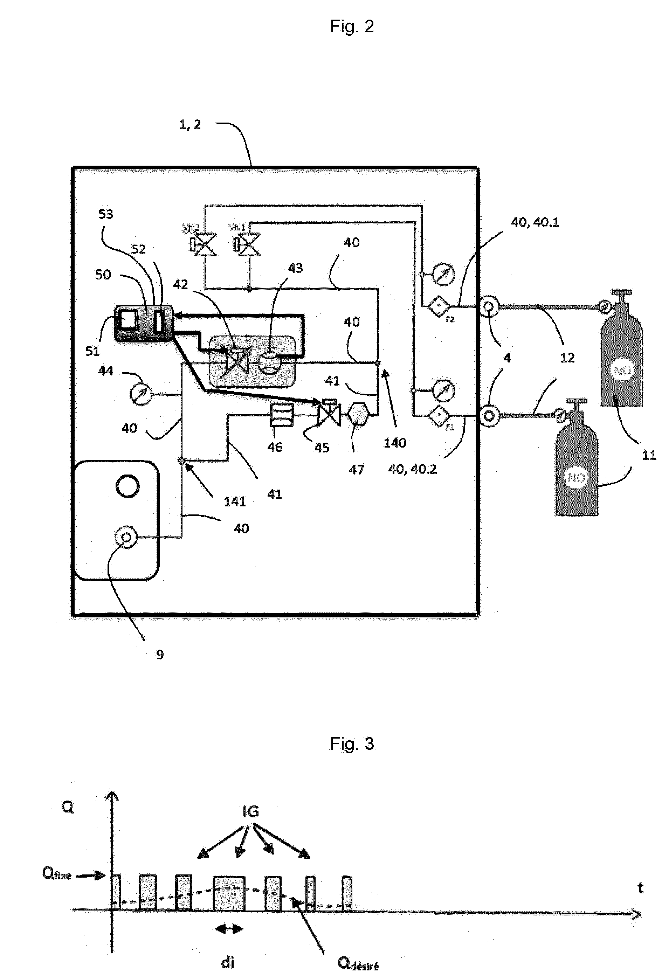

Fig. 1 schématise un mode de réalisation d'une installation 10 d'administration de gaz thérapeutique, à savoir de mélange gazeux à base de NO, à un patient comprenant un dispositif de fourniture de NO 1 selon la présente invention, en particulier celui schématisé en Fig. 2.

L'installation 10 comprend ici deux sources de gaz qui sont deux récipients 11 de gaz sous pression, à savoir des bouteilles de gaz comprimé, agencés en parallèle, contenant chacun un mélange gazeux de NO et d'azote (N₂), i.e. un mélange NO/N₂, contenant typiquement de 225 à 2000 ppmv de NO et de l'azote (N₂) pour le reste, de préférence de 450 à 1000 ppmv de NO, conditionné à une pression pouvant atteindre 150 bar, voire 180 ou plus (récipient plein), par exemple un mélange NO/N₂ contenant 450 ppmv ou 800 ppmv de NO.

Les récipients 11 de NO fournissent le mélange NO/N₂ à un dispositif ou appareil de fourniture de NO 1 selon l'invention dont une partie de l'architecture interne est schématisée sur Fig. 2. Le raccordement est assuré par des lignes 12 d'amenée de NO, i.e. des canalisations de gaz, tels des tuyaux flexibles ou analogue. Chaque ligne 12 est reliée à un port d'entrée de NO 4 du dispositif de fourniture de NO 1 pour alimenter le circuit principal de gaz 40, 40.1, 40.2 interne au boîtier 2 du dispositif de fourniture de NO 1 (cf. Fig. 2).

Le dispositif de fourniture de NO 1 comprend aussi un port d'entrée d'oxygène 5 relié fluidiquement, via une ligne d'amenée d'oxygène 14, tel un tuyau flexible ou analogue, à une source d'oxygène (non montrée), par exemple un récipient d'oxygène sous pression, typiquement une bouteille d'O₂ ou, alternativement, le réseau hospitalier, i.e. une canalisation d'amenée d'oxygène agencée dans le bâtiment hospitalier où est soigné le patient.

Les bouteilles de NO 11 et la source d'O₂, e.g. une bouteille d'oxygène, sont préférentiellement équipées d'un robinet de distribution de gaz 13, de préférence intégrant des moyens de détente de gaz, c'est-à-dire un RDI ou robinet à détendeur intégré, ou d'autres systèmes de contrôle et/ou régulation du gaz de manière à pouvoir contrôler le débit et/ou la pression du gaz qu'elles délivrent, par exemple pour fournir un mélange NO/N₂ et/ou de l'oxygène à une pression comprise entre 3 et 6 bar. Le robinet de distribution de gaz 13 peut être protégé contre les chocs par un capotage de protection (non montré).

Par ailleurs, l'installation 10 comprend aussi un ventilateur médical 30, c'est-à-dire un appareil d'assistance respiratoire, fournissant un flux de gaz respiratoire contenant de l'oxygène en quantité non-hypoxique au patient P, c'est-à-dire au moins 21% vol. d'oxygène environ, typiquement de l'air, de l'oxygène ou un mélange oxygène/azote (N₂/O₂).

Le ventilateur médical 30 est fluidiquement relié au patient via un circuit de gaz respiratoire 20 qui est ici à deux branches respiratoires 21, 22 étant donné qu'il comprend une branche inspiratoire 21, c'est-à-dire une ligne d'alimentation en gaz, servant à amener le gaz respiratoire au patient et une branche expiratoire 22 sevrant à récupérer le gaz enrichi en CO₂ expiré par le patient.

Les deux branches inspiratoire et expiratoire 21, 22 comprennent typiquement des tuyaux flexibles en polymère ou analogue. La branche inspiratoire 21 est reliée fluidiquement, en amont, à une sortie de gaz 31 du ventilateur médical 30 et, en aval, à une pièce de jonction 23, typiquement une pièce en Y. De façon analogue, la branche expiratoire 22 est reliée fluidiquement, en amont, à la pièce de jonction 23 et, en aval, à une entrée de gaz 32 du ventilateur médical 30. Les deux branches inspiratoire et expiratoire 21, 22 sont donc reliées l'une à l'autre au niveau de la pièce de jonction 23, typiquement une pièce en Y, laquelle est elle-même en communication fluidique avec une interface respiratoire 28 fournissant le gaz au patient, telle une sonde d'intubation trachéale, un masque respiratoire ou autre, de préférence une sonde d'intubation trachéale.

Bien entendu, le ventilateur médical 30 et le dispositif ou appareil de fourniture de NO 1 sont normalement alimentés électriquement par une (des) source de courant électrique, en particulier leurs composants nécessitant de l'énergie électrique pour fonctionner, en particulier les moyens de pilotage 50 et l'écran 3 du dispositif de fourniture de NO 1, le système de commande (non montré) du ventilateur médical 30, i.e. carte électronique à microprocesseur(s), ou tout autre composant, notamment la turbine interne motorisée qui fournit le flux d'air ou analogue, i.e. le gaz respiratoire. La source de courant électrique peut être le secteur (110/220V) et/ou une batterie électrique, de préférence rechargeable.

Comme on le voit, le dispositif ou appareil de fourniture de NO 1 permet d'injecter le mélange NO/N₂ dans la branche inspiratoire 21 du circuit de gaz 20, via une ligne ou conduit d'injection de NO 6 débouchant dans la branche inspiratoire 21 en un site d'injection 25, de manière à y opérer un mélange (i.e. une dilution) du flux de NO/N₂ et du flux de gaz respiratoire contenant au moins 21% d'O₂ environ, i.e. air ou de mélange oxygène/azote, délivré par le ventilateur médical 30.

Le dispositif de fourniture de NO 1 comprend un orifice ou port de sortie 9 situé au débouché de son circuit principal de gaz 40, 40.1, 40.2 par lequel le flux de NO/N₂ sort du boitier 2 du dispositif de fourniture de NO 1 et pénètre dans le conduit d'injection de NO 6 qui est raccordé fluidiquement audit orifice ou port de sortie 9, par exemple via un connecteur ou analogue.

Le mélange gazeux thérapeutique obtenu (i.e. au site d'injection 25 et en aval de la branche inspiratoire 21), c'est-à-dire le mélange final, contient donc de l'oxygène (environ >20 à 21% vol.), généralement de l'azote, et une concentration de NO variable et ajustable, typiquement comprise entre 1 et 80 ppmv, du fait de la dilution de produisant lors du mélange des flux gazeux, voire des impuretés éventuelles inévitables, comme de l'argon par exemple, qui peuvent se trouver dans le gaz mais qui ne sont pas souhaitées, en particulier quand le flux de gaz provenant du ventilateur 30 est de l'air atmosphérique plutôt qu'un mélange O₂/N₂.

Avantageusement, on prévoit en outre un humidificateur de gaz 26 agencé sur la branche inspiratoire 21 en aval du site d'injection 25 servant à humidifier le flux de gaz thérapeutique, e.g. le mélange final NO/N₂/O₂, par addition de vapeur d'eau, avant qu'il ne soit inhalé par le patient, ce qui permet d'éviter ou limiter l'assèchement des voies respiratoires du patient pendant son traitement par inhalation du gaz. Selon un autre mode de réalisation, l'humidificateur de gaz 26 pourrait aussi être agencé en amont du site d'injection 25.

Par ailleurs, optionnellement, la branche expiratoire 22 servant à recueillir les gaz expirés riches en CO₂ peut comprendre un ou d'autres composants optionnels, comme par exemple un dispositif 29 d'élimination du CO₂ et/ou de la vapeur d'eau, i.e. un piège à CO₂ et/ou à eau, tel un bac à chaud, un filtre ou autre, permettant d'éliminer (au moins une partie) le CO₂ et/ou la vapeur d'eau présents dans les gaz expirés par le patient.

Comme visible sur Fig. 1, il est aussi prévu sur la branche inspiratoire 21, en amont du site d'injection 25, un capteur de débit 24, par exemple un capteur de débit du type massique, relié au dispositif de fourniture de NO 1, notamment aux moyens de pilotage 50 dudit dispositif de fourniture de NO 1, via une ligne de mesure de débit 7 de gaz respiratoire servant à mesurer ou déterminer le débit de gaz provenant du ventilateur 30 au sein de la branche inspiratoire 21 et de le fournir aux moyens de pilotage 50 afin que ceux-ci puissent contrôler la quantité de NO/N₂ à fournir.

En effet, déterminer le débit gazeux provenant du ventilateur 30 permet notamment de réguler le passage du NO au travers du dispositif de fourniture de NO 1, c'est-à-dire de pouvoir choisir le débit de mélange NO/N₂ à injecter en fonction de la teneur en NO désirée, de la composition du mélange NO/N₂ provenant des bouteilles et du débit de gaz (i.e. air ou air/O₂) provenant du ventilateur 30. La manière de réguler le passage du NO au travers du dispositif de fourniture de NO 1 est détaillée ci-après en référence à Fig. 2 notamment.

Par ailleurs, une ligne de prélèvement 15 de gaz relie fluidiquement le dispositif de fourniture de NO 1, via une entrée de prélèvement 8, à la branche inspiratoire 21 du circuit de gaz respiratoire 20, via un connecteur de raccordement 27, de préférence à proximité de la pièce en Y 23, par exemple à environ 10 à 20 cm en amont de la pièce en Y. Cette ligne de prélèvement 15 sert à prélever des échantillons de gaz au sein de la branche inspiratoire 21 pour vérifier leur conformité avec le mélange gazeux souhaité devant être administré au patient, notamment pour ce qui est de sa teneur en NO mais aussi en O₂, voire en espèces toxiques NO₂ ayant pu être créées par oxydation de molécules de NO par de l'oxygène.

Comme déjà dit, le mélange NO/N₂ délivré par le dispositif de fourniture de NO 1 est injecté (en 25) dans le flux respiratoire contenant au moins 21% vol. d'oxygène (e.g. air ou mélange O₂/N₂) provenant du ventilateur médical 30 avant d'être administré par inhalation au patient sous forme d'un mélange respiratoire final (i.e. mélange NO/N₂/ O₂ ou NO/N₂/air) contenant généralement quelques ppmv ou dizaines ppmv de NO (ppm en volume) et au moins environ 21% vol. d'oxygène O₂, par exemple de l'ordre de 1 à 80 ppmv de NO, le reste étant généralement essentiellement de l'azote (N₂), typiquement moins de 40 ppmv de NO, par exemple de l'ordre de 20 ppmv.

Afin de délivrer la bonne quantité de NO, il est indispensable de pouvoir ajuster la quantité, i.e. le flux, de mélange NO/N₂ traversant le dispositif de fourniture de NO 1 et en ressortant par l'orifice de sortie de NO 9 avant d'être convoyé par la ligne d'injection de NO 6 jusqu'au site d'injection 25. Cette régulation est opérée par les moyens de pilotage 50 à partir non seulement des mesures du débit du gaz (i.e. air ou mélange O₂/N₂) provenant du ventilateur 30, qui sont opérées par le capteur de débit 24 agencé sur la branche inspiratoire 21, mais aussi de la teneur ou dose de NO désirée fixée par le personnel soignant et de la composition du mélange NO/N₂ provenant des bouteilles de NO 11.

A cette fin, comme illustré sur Fig. 2, le dispositif ou appareil de fourniture 1 de NO comprend un circuit de gaz principal 40 ; 40.1, 40.2 interne, i.e. passages, conduits ou analogues, pour acheminer le mélange NO/N₂ entrant par le ou les ports ou orifices d'entrée de gaz 4 jusqu'à (un ou des) l'orifice de sortie de NO 9, auquel vient se raccordement fluidiquement le conduit d'injection de NO 6.

Dans le mode de réalisation proposé, le circuit de gaz principal 40 ; 40.1, 40.2 comprend une ligne principale de NO 40 dont la partie amont se ramifie en deux tronçons parallèles 40.1, 40.2 comprenant chacune un orifice d'entrée de gaz 4 relié fluidiquement à l'une ou l'autre des bouteilles 11 de mélange NO/N₂ qui lui fournit le mélange NO/N₂ à la pression désirée, par exemple entre 3 et 6 bar de pression.

Le circuit de gaz principal 40 ; 40.1, 40.2, en particulier la ligne principale de NO 40, comprend des moyens de contrôle 42, 43 de débit de NO/N₂ qui sont pilotés par les moyens de pilotage 50 à (micro)processeur 52 du dispositif de fourniture 1 de NO. Les moyens ou un dispositif de pilotage 50 comprennent typiquement un (ou des) (micro)processeur(s) 52 agencé(s) sur une (des) carte électronique 53, dont le fonctionnement est expliqué ci-après.

Par ailleurs, un circuit de bipasse 41, aussi appelé « circuit de secours », vient se raccorder (en 140, 141) fluidiquement au circuit de gaz principal 40 ; 40.1, 40.2, en particulier à la ligne principale de NO 40, en un site de raccordement amont 140 situé en amont desdits moyens de contrôle de débit 42, 43 et en un site de raccordement aval 141 situé en aval desdits moyens de contrôle de débit 42, 43, en considérant le sens normal de circulation du mélange NO/N₂ (des entrées 4 vers la sortie 9), de manière à bipasser lesdits moyens de contrôle de débit 42, 43.

Autrement dit, le circuit de bipasse 41 comprend ou constitue une ligne de dérivation permettant de dévier le mélange NO/N₂ et éviter qu'il ne traverse les moyens de contrôle de débit 42, 43 agencés sur le circuit de gaz principal 40 ; 40.1, 40.2, en particulier sur la ligne principale de NO 40. Ce circuit de bipasse ou de secours 41 est utilisé en cas de dysfonctionnement notamment de l'électrovanne principale 42 agencée sur le circuit de gaz principal 40 ; 40.1, 40.2, afin d'assurer une délivrance de mélange NO/N₂ malgré ce dysfonctionnement.

Le circuit de bipasse 41, i.e. passage de gaz, conduit de gaz ou analogue, comprend quant à lui des moyens de contrôle de débit secondaires 45, 46, i.e. un dispositif de contrôle de débit secondaire, comprenant une (ou des) électrovanne secondaire 45, ainsi qu'un dispositif à débit fixe 46, c'est-à-dire délivrant ou fournissant (en aval) un débit de gaz fixe (Q_{fixe}) prédéterminé. Optionnellement, le débit pourrait être modifiable, notamment grâce à l'utilisation d'un dispositif détendeur 47, comme expliqué ci-après.

Selon l'invention, l'électrovanne secondaire 45 est une électrovanne de type tout ou rien (TOR), c'est-à-dire qui est configurée pour n'adopter que deux positions de fonctionnement (i.e. ouverture/fermeture), à savoir une « position ouverte » laissant passer tout le flux de mélange NO/N₂ et une « position fermée » interdisant tout passage de flux de mélange NO/N₂. A titre d'exemple, on peut utiliser l'électrovanne TOR de référence commerciale FAS 10mm PICOSOL en tant qu'électrovanne TOR 45.

Autrement, lorsque l'électrovanne 45 de type tout ou rien (TOR), i.e. l'électrovanne secondaire 45, est en position d'ouverture dite « position ouverte », l'intégralité du flux gazeux de mélange NO/N₂ peut la traverser et circuler librement dans le circuit de bipasse 41 dans le sens normal de circulation du gaz, c'est-à-dire dans le sens allant du site de raccordement amont 140 vers le site de raccordement aval 141.

A l'inverse, lorsque l'électrovanne TOR, i.e. l'électrovanne secondaire 45, est en position de fermeture dite « position fermée », le flux gazeux de mélange NO/N₂ est interrompu et ne peut plus la traverser puisqu'il est stoppé par cette électrovanne 45. La circulation libre du gaz dans le circuit de bipasse 41 est empêchée, stoppée ou impossible.

L'électrovanne secondaire 45 est commandée par les moyens de pilotage 50 à processeur 52 de manière à la faire passer d'une position à l'autre, de préférence pour opérer une délivrance de NO par pulsations ou pulses, comme détaillé ci-dessous.

Par ailleurs, un dispositif à débit fixe 46, typiquement un dispositif à orifice calibré, est agencé sur ledit circuit de bipasse 41. Il permet de délivrer un débit fixe Q_{fixe} prédéterminé de mélange gazeux NO/N₂, c'est-à-dire un débit connu, par exemple un débit de 0.1 à 2 L/min, par exemple de l'ordre de 0.5 L/min. Il est agencé en aval de l'électrovanne secondaire 45, c'est-à-dire entre l'électrovanne secondaire 45 et le site de raccordement aval 141. Le débit gazeux fixe Q_{fixe} résultant du passage du flux gazeux dans le dispositif à débit fixe 46 est connu et mémorisé dans des moyens de mémorisation 51 configurés pour mémoriser le débit fixe Q_{fixe}, de préférence les moyens de mémorisation 51 sont intégrés aux moyens de pilotage 50, notamment portés par la carte électronique 53. Les moyens de mémorisation 51 comprennent une (des) mémoire informatique, telle une mémoire flash ou autre.

Préférentiellement, le circuit de bipasse 41 peut comprendre aussi un dispositif détendeur de gaz 47 agencé en amont de l'électrovanne secondaire 45 permettant de régler ou ajuster la pression du gaz, typiquement de la réduire si nécessaire, et/ou le débit de gaz.

Dans tous les cas, les moyens de mémorisation 51 coopèrent avec les moyens de pilotage 50, en particulier avec le (les) processeur 52, pour leur fournir notamment la valeur mémorisée de débit gazeux fixe Q_{fixe}. Les moyens de mémorisation 51 utilisent la (i.e. au moins une valeur) valeur mémorisée de débit gazeux fixe Q_{fixe} pour opérer des calculs, notamment du temps d'impulsion (di) comme expliqué ci-après lorsqu'il convient de faire varier la quantité de NO fournie.

En outre, les moyens de contrôle de débit 42, 43 comprennent une électrovanne principale 42 de type proportionnelle et un capteur de débit 43, i.e. des moyens ou un dispositif de mesure de débit, agencés sur le circuit de gaz principal 40 ; 40.1, 40.2, typiquement sur la ligne principale de NO 40 dudit circuit de gaz principal 40 ; 40.1, 40.2. A titre d'exemple, on peut utiliser l'électrovanne proportionnelle de référence commerciale FAS 16mm FLATPROP en tant qu'électrovanne proportionnelle 42.

Le capteur de débit 43 est agencé préférentiellement en amont de l'électrovanne proportionnelle 42, en considérant le sens de circulation du gaz du/des orifices d'entrée 4 vers l'orifice de sortie 9, c'est-à-dire du site de raccordement amont 140 vers l'électrovanne proportionnelle 42. Le capteur de débit 43 et l'électrovanne proportionnelle 42 sont raccordés électriquement aux moyens de pilotage 50.

Le capteur de débit 43 fournit aux moyens de pilotage 50, des mesures représentatives du débit gazeux dans la ligne principale de NO 40 du circuit de gaz principal 40 ; 40.1, 40.2, immédiatement en amont de l'électrovanne proportionnelle 42. Leur fonctionnement précis est expliqué ci-après.

D'une façon générale, les moyens de pilotage 50 sont configurés pour commander l'électrovanne principale 42 et l'électrovanne secondaire 45 afin de contrôler le débit de mélange NO/N₂ traversant ces électrovannes principale 42 et secondaire 45, en particulier pour permettre au flux gazeux de circuler au travers de l'une ou l'autre de ces électrovannes principale 42 (en fonctionnement normal) et secondaire 45 (en fonctionnement de secours, i.e. en cas de dysfonctionnement) mais jamais en même temps dans les deux. Autrement dit, les deux électrovannes principale 42 et secondaire 45 ne sont jamais ouvertes simultanément de sorte que le flux gazeux doive obligatoirement circuler de manière exclusive dans l'une des deux.

Les composants de l'appareil 1 sont agencés dans un boitier 2, c'est-à-dire une carcasse externe rigide.

En temps normal, c'est-à-dire lors d'un fonctionnement normal de l'appareil de délivrance de NO 1, le flux gazeux de mélange gazeux NO/N₂ circule dans le circuit principal de gaz 40, 40.1, 40.2, en particulier dans la ligne principale de NO 40, et traverse l'électrovanne principale 42 puisque cette électrovanne principale 42 est ouverte pour laisser circuler le gaz dans le circuit principal de gaz 40, 40.1, 40.2, en particulier dans la ligne principale de NO 40, alors que l'électrovanne secondaire 45 est fermée pour empêcher toute circulation de gaz dans le circuit de bipasse 41 qui comprend l'électrovanne secondaire 45.

A l'inverse, en cas de problème, par exemple en cas de défaut du capteur de débit 43 ou de dysfonctionnement de l'électrovanne principale 42, le flux gazeux de mélange gazeux NO/N₂ est dévié vers le circuit de bipasse 41, qui fait alors office de circuit de secours. L'électrovanne principale 42 passe alors en position fermée pour stopper/empêcher toute circulation du flux gazeux au travers l'électrovanne principale 42, c'est-à-dire dans la portion de ligne principale de NO 40 comprenant les moyens de contrôle de débit 42, 43, y compris l'électrovanne principale 42, laquelle est située schématiquement entre les sites de raccordement amont 140 et aval 141.

Afin de pouvoir mieux contrôler le débit du mélange gazeux NO/N₂ fourni par l'appareil de délivrance de NO 1, c'est-à-dire délivré notamment par l'orifice ou port de sortie 9 qui est situé au débouché de son circuit principal de gaz 40, 40.1, 40.2, lorsque ce flux gazeux circule dans le circuit de bipasse 41, c'est-à-dire en mode secours, l'électrovanne secondaire 45 qui est une électrovanne de type tout ou rien (TOR) est contrôlée en mode pulsé par les moyens de pilotage 50, c'est-à-dire qu'elle ne délivre pas un flux continu de NO/N₂ mais des petites doses ou impulsions (i.e. pulses) de NO/N₂ gazeux.

Lorsque les moyens de pilotage 50 pilotent l'électrovanne secondaire 45 pour délivrer des impulsions gazeuses ou « pulses » de gaz dans le circuit de bipasse 41, i.e. en aval de l'électrovanne secondaire 45, l'électrovanne principale 42 est quant à elle fermée pour empêcher sa traversée par le flux gazeux, i.e. le flux de mélange NO/N₂.

Plus précisément, les moyens de pilotage 50 sont configurés, e.g. programmés, pour commander l'électrovanne secondaire 45 en mode pulsé de manière à la faire passer alternativement, i.e. au fil du temps, en position d'ouverture et en position de fermeture et à délivrer ainsi le mélange NO/N₂ sous forme d'impulsions gazeuses successives, i.e. de « pulses » de gaz.

On peut contrôler les impulsions gazeuses. En effet, chaque impulsion gazeuse comprend ou se caractérise par un temps d'impulsion (di) de durée non-nulle (i.e. > 0 msec) pendant lequel l'électrovanne secondaire 45 est en position d'ouverture et autorise ainsi le passage d'une quantité de mélange NO/N₂ souhaitée, c'est-à-dire sa traversée par une quantité de gaz donnée, en direction du dispositif à débit fixe 46 agencé sur ledit circuit de bipasse 41, en aval de l'électrovanne secondaire 45 qui est une électrovanne TOR commandée par les moyens de pilotage 50.

La quantité de mélange NO/N₂ souhaitée est donc variable puisqu'elle correspond au débit fixe (Q_{fixe}) prédéterminé de mélange NO/N₂ délivré par le dispositif à débit fixe 46 pendant le temps d'impulsion (di) de l'impulsion gazeuse considérée, c'est-à-dire le temps pendant lequel l'électrovanne secondaire 45 est en position d'ouverture et autorise le passage de gaz.

Autrement dit, la proportion ou quantité de mélange NO/N₂ traversant l'électrovanne secondaire 45 de type TOR peut être contrôlée en jouant sur le temps d'impulsion (di) de chaque impulsion gazeuse considérée puisque le dispositif à débit fixe 46 délivre un débit fixe (Q_{fixe}) prédéterminé, i.e. connu, de mélange NO/N₂.

Le temps d'impulsion (di) de chaque impulsion gazeuse est quant à lui calculé ou déterminé par les moyens de pilotage 50 en fonction notamment du débit de gaz désiré (Q_{désiré}) mais aussi de la teneur en NO du mélange NO/N₂ et d'un (ou plusieurs) débit prédéfini et généralement mémorisé, en particulier en cas de défaut du capteur 24.

Ainsi, Fig. 3 schématise une pluralité d'impulsions gazeuses IG successives obtenues au fil du temps (t) par contrôle de l'ouverture/fermeture de l'électrovanne secondaire 45 de type TOR en mode pulsé.

On voit que chaque impulsion gazeuse IG a une durée ou temps d'impulsion (di) non-nul (i.e. > 0 msec) mais variable de manière à délivrer des doses ou quantités de mélange NO/N₂ variables et obtenir ainsi un débit de gaz désiré (Q_{désiré}) en aval du dispositif à débit fixe 46. Typiquement, les temps d'impulsion (di) peuvent atteindre environ 200 msec.

Par ailleurs, selon l'invention, en fonctionnement normal (i.e. hors tout dysfonctionnement), lorsque ce flux gazeux ne circule pas dans le circuit de bipasse 41, le contrôle du flux traversant l'électrovanne proportionnelle 42 agencée sur le circuit principal de gaz 40, 40.1, 40.2 se fait de manière proportionnelle.

D'une façon générale, l'électrovanne proportionnelle 42 est configurée pour présenter des degrés d'ouverture allant de 0 à 100% où :
- 0% correspond à une fermeture totale de l'électrovanne 42 empêchant tout passage de flux gazeux,
- 100% correspond à une ouverture totale de l'électrovanne 42 autorisant un débit maximum du flux gazeux, et
- les valeurs intermédiaires comprises entre 0 et 100% correspondent à des degrés d'ouverture intermédiaires, c'est-à-dire des ouvertures partielles de l'électrovanne 42 autorisant le passage de débits gazeux non-nuls mais inférieurs au débit maximum du flux gazeux.

Dans ce cas, les moyens de pilotage 50 sont configurés pour commander l'électrovanne proportionnelle 42 de manière différente selon que cette électrovanne proportionnelle 42 se trouve dans sa plage de fonctionnement linéaire ou dans sa plage de fonctionnement non-linéaire.

En effet, l'électrovanne proportionnelle 42 est configurée pour fonctionner dans (au moins) deux plages de fonctionnement données comprises entre 0 et 100%, à savoir :
- une première plage de fonctionnement, i.e. non-linéaire, aussi dite « basse » correspondant à des degrés d'ouverture de l'électrovanne proportionnelle 42 compris entre 0 et x% avec : 0 < x% ≤ 10%, de préférence 0 < x% ≤ 5%, par exemple entre 0 et 1% (i.e. x% = 1%), et
- une deuxième plage de fonctionnement, i.e. linéaire, correspondant à des degrés d'ouverture supérieurs à x%, i.e. entre x% et au moins 85%, par exemple compris entre x% et 90%, par exemple entre 1 et 90%, typiquement entre 10 et 90% de sa plage de fonctionnement.

Optionnellement, l'électrovanne proportionnelle 42 peut être configurée pour fonctionner selon une troisième plage de fonctionnement, i.e. non-linéaire, aussi dite « haute » correspondant à des degrés d'ouverture proches de 100%, par exemple d'au moins 80%, de préférence d'au moins 90%, par exemple entre 90% et 100%.

Dans tous les cas, selon l'invention, les moyens de pilotage 50 sont configurés pour commander l'électrovanne proportionnelle 42 pour opérer une fourniture de gaz, c'est-à-dire de mélange NO/N₂, de manière différente selon la plage de fonctionnement considérée, c'est-à-dire selon le degré d'ouverture de l'électrovanne proportionnelle 42, à savoir :
- en mode proportionnel dans la deuxième plage de fonctionnement, i.e. plage linéaire, c'est-à-dire pour les degrés d'ouverture supérieurs à x%, typiquement entre 1 et 90%, par exemple entre 1% et 90% (x%=1%) ou entre 10% et 90% (x%=10%), et
- en mode pulsé ou en modulation d'impulsions dans la première plage de fonctionnement, i.e. plage non-linéaire, c'est-à-dire entre 0 et x%, avec x% ≤ 10%, par entre 0 et 10% (x%=10%) ou entre 0 et 1% (x%=1%).

Bien entendu, x% peut revêtir des valeurs entre 1 et 10%, par exemple 2%, 3%, 4%, 5%, 7%... ou autre.

Dès lors, il apparaît que :
- en mode proportionnel, le flux gazeux traversant l'électrovanne 42 est continu
- en mode pulsé, le flux gazeux traversant l'électrovanne 42 prend la forme d'impulsions gazeuses (IG) successives, donc est discontinu.

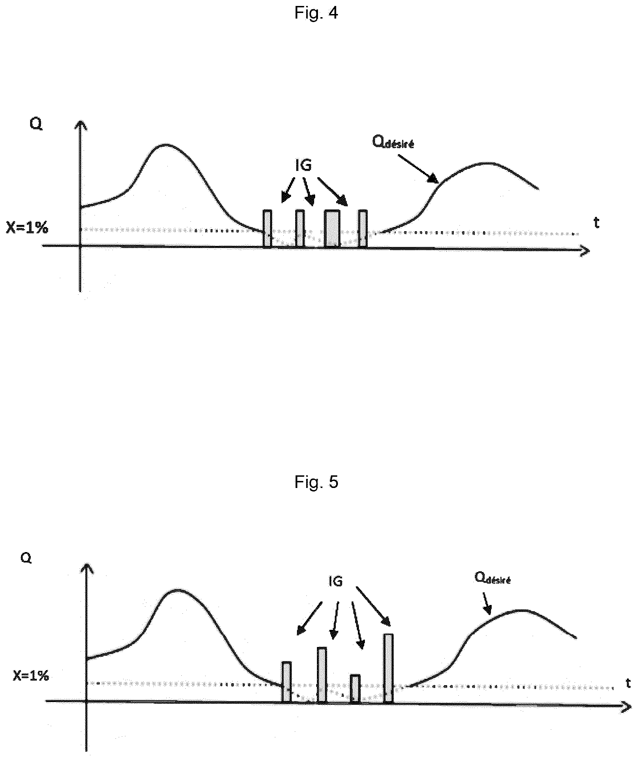

Ceci est illustré en Fig. 4 et Fig. 5, où l'on a schématisé les impulsions IG ou pulses gazeux délivrés par l'électrovanne proportionnelle 42 de manière à obtenir le débit de gaz désiré (Q_{désiré}) en aval de l'électrovanne proportionnelle 42.

Sur ces Fig. 4&5, la première plage de fonctionnement qui est non-linéaire va de 0 à 1 % (x%=1%) et la deuxième plage de fonctionnement qui est linéaire va de 1 à 90% environ.

En Fig. 4, on voit qu'il est possible de fixer, ajuster ou moduler le débit de gaz fourni en jouant sur la largeur de chaque impulsion gazeuse IG (i.e. largeur des IG sur le graphe) qui correspond au temps (i.e. la durée) d'ouverture de l'électrovanne proportionnelle 42. En effet, plus le temps d'ouverture de l'électrovanne proportionnelle 42 est important, plus la quantité de gaz (i.e. débit) qui peut la traverser, lors de chaque impulsion IG, est élevée, et inversement.

Par ailleurs, en Fig. 5, on voit qu'il est aussi possible de fixer, ajuster ou moduler le débit de gaz fourni en jouant sur l'amplitude de chaque impulsion gazeuse IG (i.e. sa hauteur des IG sur le graphe), laquelle correspond au degré d'ouverture de l'électrovanne proportionnelle 42. En effet, plus le degré d'ouverture (x%) de l'électrovanne proportionnelle 42 est élevé (par exemple proche de 1% quand x=1%), plus son ouverture/sa section de passage est grande, donc plus de gaz peut la traverser lors de chaque impulsion IG, et inversement.

En faisant varier les amplitudes et/ou les largeurs des impulsions gazeuses, on peut délivrer une quantité de NO qui correspond au débit de gaz désiré (Q_{désiré}).

Ce contrôle d'amplitude ou de durée des impulsions gazeuses IG, est opéré par les moyens de pilotage 50 qui pilotent l'électrovanne proportionnelle 42, notamment en fonction du débit de gaz désiré, lequel dépend du débit du flux de gaz contenant de l'oxygène (> env. 20%vol) délivré par le ventilateur 30 mesuré par le capteur de débit 24, tel un flux d'air ou de mélange gazeux O₂/N₂, de la concentration de NO dans le mélange gazeux NO/N₂ alimentant l'appareil 1 et de la posologie souhaitée, c'est-à-dire de la teneur en NO souhaitée dans le mélange gazeux final résultant du mélange du flux de gaz contenant de l'oxygène (> env. 20%vol) délivré par le ventilateur 30 et du flux de NO/N₂ provenant de l'appareil 1.

L'appareil de fourniture de NO 1 et/ou l'installation de fourniture de gaz de l'invention 10 servent à opérer une administration par inhalation, en particulier via une sonde d'intubation trachéale, d'un mélange gazeux comprenant de 1 à 80 ppmv de NO, typiquement de l'ordre de 10 à 20 ppmv de NO, et au moins 20%vol. environ d'oxygène à un patient en ayant besoin (e.g. adulte, enfant, adolescent ou nouveau-né, y compris un prématuré), typiquement un patient (i.e. une personne humaine) souffrant d'hypertension pulmonaire et/ou d'hypoxie susceptibles d'engendrer des vasoconstrictions pulmonaires ou analogues, par exemple causés par des pathologies ou troubles pulmonaires de type PPHN (hypertension pulmonaire persistante du nouveau-né) ou SDRA (syndrome de détresse respiratoire aigüe), ou encore engendrés par une opération de chirurgie cardiaque avec mise du patient sous circulation sanguine extracorporelle (CEC).

## Revendications

1. Appareil de fourniture de NO (1) comprenant :
- un circuit de gaz principal (40 ; 40.1, 40.2) pour acheminer un mélange gazeux NO/N₂,
- des moyens de contrôle de débit (42, 43) comprenant une électrovanne principale (42) agencée sur le circuit de gaz principal (40 ; 40.1, 40.2), et
- des moyens de pilotage (50) configurés pour commander au moins ladite électrovanne principale (42) pour contrôler le débit de mélange gazeux NO/N₂ traversant ladite électrovanne principale (42),
**caractérisé en ce que** :
- l'électrovanne principale (42) est une électrovanne proportionnelle ayant des degrés d'ouverture compris entre 0 et 100%, et est configurée pour fonctionner dans au moins deux plages de fonctionnement données comprenant au moins :
▪ une première plage de fonctionnement correspondant à des degrés d'ouverture de l'électrovanne principale (42) compris entre 0 et x% avec : 0 < x% ≤ 10%, et
▪ une deuxième plage de fonctionnement correspondant à des degrés d'ouverture de l'électrovanne principale (42) > x%, et
- les moyens de pilotage (50) sont configurés pour commander ladite électrovanne principale (42) pour opérer une fourniture de gaz de manière différente selon la plage de fonctionnement considérée, à savoir :
▪ en mode pulsé dans la première plage de fonctionnement de manière à délivrer des impulsions (IG) gazeuses successives, et
▪ en mode proportionnel dans la deuxième plage de fonctionnement,
- et il comprend en outre un circuit de bipasse (41) venant se raccorder fluidiquement au circuit de gaz principal (40 ; 40.1, 40.2), en amont et en aval des moyens de contrôle de débit (42, 43) et comprenant des moyens de contrôle de débit secondaires (45, 46) comprenant une électrovanne secondaire (45) de type tout ou rien et un dispositif à débit fixe (46).

2. Appareil selon la revendication 1, **caractérisé en ce que** les moyens de pilotage (50) comprennent au moins un microprocesseur agencé sur une carte électronique.

3. Appareil selon la revendication 1, **caractérisé en ce que** la première plage de fonctionnement correspondant à des degrés d'ouverture de l'électrovanne principale (42) compris entre 0 et x% avec : 0 < x% ≤ 5%.

4. Appareil selon la revendication 1, **caractérisé en ce que** les moyens de pilotage (50) sont configurés pour fixer ou ajuster une durée ou une amplitude de chaque impulsion (IG) gazeuse délivrée en mode pulsé, pour contrôler le débit de gaz traversant ladite électrovanne principale (42).

5. Appareil selon la revendication 4, **caractérisé en ce que** les moyens de pilotage (50) sont configurés pour ajuster, pour chaque impulsion, la durée d'impulsion (IG) en contrôlant la durée d'ouverture de l'électrovanne proportionnelle (42).

6. Appareil selon la revendication 4, **caractérisé en ce que** les moyens de pilotage (50) sont configurés pour ajuster, pour chaque impulsion, la durée d'impulsion (IG) en contrôlant l'amplitude de chaque impulsion (IG) en contrôlant le degré d'ouverture (x%) de l'électrovanne proportionnelle (42).

7. Appareil selon la revendication 5, **caractérisé en ce que** la durée d'ouverture de l'électrovanne proportionnelle (42) est comprise entre 5 et 200 msec.

8. Appareil selon la revendication 5 ou 6, **caractérisé en ce qu'**en mode pulsé, les moyens de pilotage (50) sont configurés pour commander l'électrovanne proportionnelle (42) pour délivrer des impulsions gazeuses successives où chaque impulsion gazeuse présente une durée d'impulsion (di) non-nulle pendant laquelle l'électrovanne proportionnelle (42) est en position d'ouverture partielle avec un degré d'ouverture x%, tel que x%<10%, de sorte d'autoriser le passage d'une quantité de mélange NO/N₂ donnée pendant ladite durée d'impulsion (di).

9. Appareil selon la revendication 8, **caractérisé en ce que** le temps d'impulsion (di) de chaque impulsion gazeuse (IG) est de durée variable non-nulle.

10. Appareil selon la revendication 9, **caractérisé en ce que** le temps d'impulsion (di) de chaque impulsion gazeuse (IG) est compris entre 5 et 200 msec.

11. Appareil selon la revendication 1, **caractérisé en ce que** le dispositif à débit fixe (46) comprend un orifice calibré.

12. Appareil selon la revendication 11, **caractérisé en ce que** l'électrovanne secondaire (45) de type tout ou rien est contrôlée en mode pulsé par les moyens de pilotage (50).

13. Appareil selon la revendication 12, **caractérisé en ce que** l'électrovanne secondaire (45) est configurée pour adopter uniquement deux positions de fonctionnement comprenant une position ouverte laissant passer le flux de mélange NO/N₂ et une position fermée interdisant tout passage de flux de mélange NO/N₂.

14. Appareil selon la revendication 11, **caractérisé en ce que** le dispositif à débit fixe (46) comprend un dispositif à orifice calibré délivrant un débit fixe prédéterminé (Q_{fixe}) compris entre 0.1 et 2 L/min.

15. Installation de fourniture de NO (10) à un patient comprenant :
- au moins une source (11) de mélange NO/N₂,
- un appareil de fourniture de NO (1) selon l'une des revendications précédentes, raccordé fluidiquement à ladite au moins une source (11) de mélange NO/N₂,
- un ventilateur médical (30) configuré pour fournir un gaz respiratoire contenant de l'oxygène, typiquement au moins 20% environ d'oxygène, tel de l'air ou un mélange N₂/O₂,
- un circuit patient (20) auquel sont raccordés fluidiquement l'appareil de fourniture de NO (1) et le ventilateur médical (30) pour alimenter ledit circuit patient (20) en ledit mélange gazeux NO/N₂ et en ledit gaz respiratoire contenant de l'oxygène, respectivement, et
- un capteur de débit (24) agencé sur le circuit patient (20) et configuré pour déterminer et fournir aux moyens de pilotage (50) de l'appareil de fourniture de NO (1), au moins un signal de mesure représentatif du débit gazeux au sein du circuit patient (20).

## Patentansprüche

1. Vorrichtung zur Zufuhr von NO (1), umfassend:
- einen Hauptgaskreislauf (40; 40.1, 40.2) zum Leiten eines NO/N₂-Gasgemisches ,
- Durchflusssteuermittel (42, 43) mit einem Hauptmagnetventil (42), das in dem Hauptgasstrom (40; 40.1, 40.2) angeordnet ist, und
- Steuermittel (50), die so konfiguriert sind, dass sie mindestens das eine Hauptmagnetventil (42) steuern, um den Durchfluss des NO/N₂-Gasgemisches durch das Hauptmagnetventil (42) zu regeln,
**dadurch gekennzeichnet, dass**:
- das Hauptmagnetventil (42) ein Proportionalmagnetventil mit Öffnungsgraden zwischen 0 und 100 % ist und so konfiguriert ist, dass es in mindestens zwei vorgegebenen Betriebsbereichen arbeitet, die mindestens Folgendes umfassen:
▪ einen ersten Betriebsbereich, der Öffnungsgraden des Hauptmagnetventils (42) zwischen 0 und x % entspricht, wobei: 0 < x % ≤ 10 % und
▪ einem zweiten Betriebsbereich, der Öffnungsgraden des Hauptmagnetventils (42) von > x % entspricht, und
- die Steuermittel (50) so konfiguriert sind, dass sie das Hauptmagnetventil (42) so steuern, dass es je nach dem betrachteten Betriebsbereich eine unterschiedliche Gasversorgung bewirkt, nämlich:
▪ im Impulsmodus im ersten Betriebsbereich, um aufeinanderfolgende Gasimpulse (IG) zu liefern, und
▪ im proportionalen Modus im zweiten Betriebsbereich,
- und es umfasst außerdem einen Bypass-Kreislauf (41), der fluidisch mit dem Hauptgaskreislauf (40; 40.1, 40.2) stromaufwärts und stromabwärts der Durchflussregelungsmittel (42, 43) angeschlossen ist und sekundäre Durchflussregelungsmittel (45, 46) umfasst, die ein sekundäres Magnetventil (45) vom Typ "alles oder nichts" und eine Vorrichtung mit festem Durchfluss (46) umfassen.

2. Vorrichtung nach Anspruch 1, **dadurch gekennzeichnet, dass** die Steuermittel (50) mindestens einen auf einer Elektronikkarte angeordneten Mikroprozessor umfassen.

3. Vorrichtung nach Anspruch 1, **dadurch gekennzeichnet, dass** der erste Betriebsbereich Öffnungsgraden des Hauptmagnetventils (42) zwischen 0 und x % entspricht, wobei: 0 < x % ≤ 5 %.

4. Vorrichtung nach Anspruch 1, **dadurch gekennzeichnet, dass** die Steuermittel (50) so konfiguriert sind, dass sie eine Dauer oder Amplitude jedes im Impulsmodus abgegebenen Gasimpulses (IG) festlegen oder einstellen, um den Gasdurchfluss durch das Hauptmagnetventil (42) zu steuern.

5. Vorrichtung nach Anspruch 4, **dadurch gekennzeichnet, dass** die Steuermittel (50) so konfiguriert sind, dass sie für jeden Impuls die Impulsdauer (IG) durch Steuern der Öffnungsdauer des Proportionalmagnetventils (42) einstellen.

6. Vorrichtung nach Anspruch 4, **dadurch gekennzeichnet, dass** die Steuermittel (50) so konfiguriert sind, dass sie für jeden Impuls die Impulsdauer (IG) durch Steuern der Amplitude jedes Impulses (IG) durch Steuern des Öffnungsgrades (x %) des Proportionalmagnetventils (42) einstellen.

7. Vorrichtung nach Anspruch 5, **dadurch gekennzeichnet, dass** die Öffnungsdauer des Proportionalmagnetventils (42) zwischen 5 und 200 ms liegt.

8. Vorrichtung nach Anspruch 5 oder 6, **dadurch gekennzeichnet, dass** im Impulsmodus die Steuermittel (50) so konfiguriert sind, dass sie das Proportionalmagnetventil (42) so steuern, dass es aufeinanderfolgende Gasimpulse abgibt, wobei jeder Gasimpuls eine (di) ungleich Null aufweist, während der sich das Proportionalmagnetventil (42) in einer teilweise geöffneten Position mit einem Öffnungsgrad x % befindet, wobei x % < 10 % ist, so dass der Durchfluss einer bestimmten Menge des NO/N(₂)-Gemischs während der genannten Impulsdauer (di) ermöglicht wird.

9. Vorrichtung nach Anspruch 8, **dadurch gekennzeichnet, dass** die Impulszeit (di) jedes Gasimpulses (IG) eine variable Dauer ungleich Null hat.

10. Vorrichtung nach Anspruch 9, **dadurch gekennzeichnet, dass** die Impulsdauer (di) jedes Gasimpulses (IG) zwischen 5 und 200 ms liegt.

11. Vorrichtung nach Anspruch 1, **dadurch gekennzeichnet, dass** die Vorrichtung mit festem Durchfluss (46) eine kalibrierte Öffnung umfasst.

12. Vorrichtung nach Anspruch 11, **dadurch gekennzeichnet, dass** das sekundäre Magnetventil (45) vom Typ "alles oder nichts" durch die Steuermittel (50) im Impulsmodus gesteuert wird.

13. Vorrichtung nach Anspruch 12, **dadurch gekennzeichnet, dass** das sekundäre Magnetventil (45) so konfiguriert ist, dass es nur zwei Betriebspositionen einnehmen kann, darunter eine geöffnete Position, die den Durchfluss des NO/N₂-Gemisches ermöglicht, und eine geschlossene Position, die jeglichen Durchfluss des NO/N₂-Gemisches verhindert.

14. Vorrichtung nach Anspruch 11, **dadurch gekennzeichnet, dass** die Vorrichtung mit festem Durchfluss (46) eine kalibrierte Öffnungsvorrichtung umfasst, die einen vorbestimmten festen Durchfluss (Q_{fest} ) zwischen 0,1 und 2 I/min liefert.

15. Anlage zur Zufuhr von NO (10) zu einem Patienten, umfassend:
- mindestens eine Quelle (11) für ein NO/N₂-Gemisch ,
- eine Vorrichtung zur Zufuhr von NO (1) gemäß einem der vorstehenden Ansprüche, das fluidisch mit der mindestens einen Quelle (11) für ein NO/N₂-Gemisch verbunden ist,
- ein medizinisches Beatmungsgerät (30), das so konfiguriert ist, dass es ein sauerstoffhaltiges Atemgas, typischerweise mit mindestens etwa 20 % Sauerstoff, wie Luft oder ein N₂/O₂-Gemisch,
- einen Patientenkreislauf (20), mit der Vorrichtung zur Zufuhr von NO (1) und das medizinische Beatmungsgerät (30) fluidisch verbunden sind, um den Patientenkreislauf (20) mit dem NO/N₂-Gasgemisch bzw. dem sauerstoffhaltigen Atemgas zu versorgen, und
- ein Durchflusssensor (24), der am Patientenkreislauf (20) angeordnet und so konfiguriert ist, dass er mindestens ein Messsignal, das den Gasdurchfluss im Patientenkreislauf (20) repräsentiert, ermittelt und an die Steuereinrichtung (50) der NO-Zufuhrvorrichtung (1) liefert.

## Claims

1. A NO supply apparatus (1) comprising:
- a main gas circuit (40; 40.1, 40.2) for conveying an NO/N 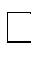 gas mixture,
- flow control means (42, 43) comprising a main solenoid valve (42) arranged on the main gas circuit (40; 40.1, 40.2), and
- control means (50) configured to control at least said one main solenoid valve (42) to control the flow rate of NO/N□ gas mixture passing through said main solenoid valve (42),
**characterized in that**:
- the main solenoid valve (42) is a proportional solenoid valve having opening degrees between 0 and 100%, and is configured to operate in at least two given operating ranges comprising at least:
▪ a first operating range corresponding to degrees of opening of the main solenoid valve (42) between 0 and x% with: 0 < x% ≤ 10%, and
▪ a second operating range corresponding to opening degrees of the main solenoid valve (42) > x%, and
- the control means (50) are configured to control said main solenoid valve (42) to operate a gas supply differently according to the operating range in question, namely:
▪ in pulse mode in the first operating range so as to deliver successive gas pulses (IG), and
▪ in proportional mode in the second operating range,
- and it further comprises a bypass circuit (41) fluidly connected to the main gas circuit (40; 40.1, 40.2), upstream and downstream of the flow control means (42, 43) and comprising secondary flow control means (45, 46) comprising a secondary solenoid valve (45) of the on/off type and a fixed flow device (46).

2. Apparatus according to claim 1, **characterized in that** the control means (50) comprise at least one microprocessor arranged on an electronic card.

3. Apparatus according to claim 1, **characterized in that** the first operating range corresponds to degrees of opening of the main solenoid valve (42) between 0 and x%, where: 0 < x% ≤ 5%.

4. Apparatus according to claim 1, **characterized in that** the control means (50) are configured to set or adjust a duration or amplitude of each gas pulse (IG) delivered in pulse mode, to control the gas flow rate through said main solenoid valve (42).

5. Apparatus according to claim 4, **characterized in that** the control means (50) are configured to adjust, for each pulse, the pulse duration (IG) by controlling the opening duration of the proportional solenoid valve (42).

6. Apparatus according to claim 4, **characterized in that** the control means (50) are configured to adjust, for each pulse, the pulse duration (IG) by controlling the amplitude of each pulse (IG) by controlling the degree of opening (x%) of the proportional solenoid valve (42).

7. Apparatus according to claim 5, **characterized in that** the opening duration of the proportional solenoid valve (42) is between 5 and 200 msec.

8. Apparatus according to claim 5 or 6, **characterized in that**, in pulsed mode, the control means (50) are configured to control the proportional solenoid valve (42) to deliver successive gas pulses, each gas pulse having a non-zero pulse duration (di) during which the proportional solenoid valve (42) is in a partially open position with an opening degree x%, such that x% < 10%, of , so as to allow the passage of a given quantity of NO/N(₂₎mixture during said pulse duration (di).

9. Apparatus according to claim 8, **characterized in that** the pulse time (di) of each gas pulse (IG) is of variable non-zero duration.

10. Apparatus according to claim 9, **characterized in that** the pulse time (di) of each gas pulse (IG) is between 5 and 200 msec.

11. Apparatus according to claim 1, **characterized in that** the fixed flow device (46) comprises a calibrated orifice.

12. Apparatus according to claim 11, **characterized in that** the secondary solenoid valve (45) of the on/off type is controlled in pulsed mode by the control means (50).

13. Apparatus according to claim 12, **characterized in that** the secondary solenoid valve (45) is configured to adopt only two operating positions comprising an open position allowing the NO/N₂ mixture flow to pass through and a closed position preventing any passage of the NO/N₂ mixture flow .

14. Apparatus according to claim 11, **characterized in that** the fixed flow device (46) comprises a calibrated orifice device delivering a predetermined fixed flow rate (Q_{fixed} ) between 0.1 and 2 L/min.

15. Installation for providing NO (10) to a patient, comprising:
- at least one source (11) of NO/N□ mixture,
- an NO supply apparatus (1) according to one of the preceding claims, fluidly connected to said at least one source (11) of NO/N 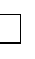 mixture,
- a medical ventilator (30) configured to supply a respiratory gas containing oxygen, typically at least approximately 20% oxygen, such as air or a N 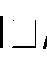 /O 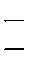 mixture,
- a patient circuit (20) to which the NO supply apparatus (1) and the medical ventilator (30) are fluidly connected to supply said patient circuit (20) with said NO/N 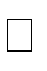 gas mixture and said oxygen-containing respiratory gas, respectively, and
- a flow sensor (24) arranged on the patient circuit (20) and configured to determine and supply to the control means (50) of the NO supply apparatus (1) at least one measurement signal representative of the gas flow within the patient circuit (20).
